# EUROPEAN PATENT SPECIFICATION

(11) **EP 1 357 882 B1**
(45) Date of publication and mention of the grant of the patent: **09.11.2005**
(21) Application number: 02710202.9
(22) Date of filing: 06.02.2002
(51) Int. Cl.: A61K 6/00

(54) **METHOD OF TOOTH WHITENING WITH SYNTHETIC RESIN IN WHITE LIQUID NAIL-POLISH TYPE**
VERFAHREN ZUR BLEICHUNG VON ZÄHNEN MIT EINER WEISSEN FLÜSSIGEN NAGELLACK ÄHNLICHEM KUNSTSTOFFZUSAMMENSETZUNG
METHODE DE BLANCHIMENT DES DENTS AVEC DE LA RESINE SYNTHETIQUE INCORPOREE DANS UN LIQUIDE BLANC DU TYPE VERNIS A ONGLES

(30) Priority: 07.02.2001 GR 2001100059
(43) Date of publication of application: 05.11.2003
(73) Proprietor: Kostomiri, Despina, 151 21 Pefki Attikis (GR)
(72) Inventor: Kostomiri, Despina, 151 21 Pefki Attikis (GR)
(74) Representative: Altenburg, Udo
(86) International application number: PCT/GR2002/000006
(87) International publication number: WO 2002/062303

(56) References cited:
- WO-A-98/46196
- WO-A-98/48766
- WO-A-99/17716
- DE-A- 4 027 265
- US-A- 4 134 935
- US-A- 5 645 429
- CHEMICAL ABSTRACTS, vol. 130, no. 7, 1999 Columbus, Ohio, US; abstract no. 86126, FRANKENBERGER,R ET ALL.: "The long-term stability of the composite/dentin bond after total etching" XP002184940 & DTSCH. ZAHNAERZTL., vol. 53, no. 10, 1998, pages 697-700, Germany

## Description

The invention refers to a method for teeth whitening, based on the utilization of white, nail-polish-type, liquid synthetic resin produced in many shades.

Tooth whitening is a known technique that is based until today on the utilization of whitening gels or laser.

Both existing methods have the following disadvantages:

They do not achieve, in all cases, the desired (preselected) whiteness of the teeth, because they remove only the acquired stains and can restore the original color in only a small percentage of people (the genetically determined color of the enamel, which usually is not white).

If there are problems with tooth fractures or old, unsightly fillings or problems with recession of the gingiva (cervical recessions) they cause sensitivities of the tooth and their application must stop immediately without the option of using them in the future.

As far as lasers and their application, special protection of the gingiva is required so as not to create health problems (in the gingiva).

The same apply to the whitening gels, but in a lesser degree.

Another disadvantage for gels is the fact that to achieve a result (if any) they have to be applied daily for several days.

It is noted that, it is not allowed to apply repeatedly and frequently whitening gels in order to preserve the shade of white, because the strength of the enamel is reduced.

The results, achieved with the gels, are not long term.

In a short time, usually a few months, the teeth return to their original color condition.

If there are white fillings of resins on the tooth, they can change only the color of the enamel and not of the filling. So after the whitening there is a difference in color between filling and enamel (unaesthetic result).

In conclusion, the techniques of using gels and lasers cannot produce a desirable (preselected) whiteness, but in the best case, the original (inherited) color of the enamel (which usually is not white).

To achieve this result, they must be applied more than once (mainly gels) and it is possible to create problems with gingiva and sensitivity to the teeth, and their results are limited in time.
**In this invention,** whitening is achieved by using a liquid, nail-polish-type, white synthetic resin with several shades of white according to the method of claim 1.

**The advantages** of tooth whitening with synthetic resin (or other dental material in liquid form) are the following:
- The tone of white we preselected is achieved with only one application.
- It is independent on the inherited color of the enamel.
- We do not have problems with sensitivity, because resins function in dental practice for therapeutic reasons (white fillings) and are recommended materials for therapeutic reasons.
- We do not have therapeutic problems with the gingiva, because they are materials, compatible with the oral cavity (are used for therapeutic reasons in the mouth).
- We have a many years, both clinical and laboratory, experience with resins, because they are used for many years in therapeutic restorations of dental fillings, having long term results.
- If there are white fillings, in the teeth, they will change the color of the filling and not only of the tooth, given the fact that they are materials, similar to that of the filling.
- Using this invention, the results of tooth whitening are long term.

The invention is described below, presenting the methodology:

### Short description

**Stage 1:** Teeth-treatment by using known methods.
**Stage 2:** Painting in a nail-polish-type of a liquid white resin (in many shades). New method, new product, new application.
**Stage 3:** Photopolymerization of the liquid white resin of many shades.

### Detailed description

### Stage 1 Teeth-treatment, by using known methods:

Indicatively, one of the most known methods of teeth-treatment, which is used for therapeutic reasons, while in this invention is for whitening, is described below.

The teeth are cleaned. We pass a transparent isolating strip to the tooth in order to check the exact limits of whitening. The enamel is etched with H3PO4 (orthophosphoric acid). We rinse with water. We dry. We apply a one-phase adhesive factor and photopolymerize).

### Stage 2 Painting in a nail-polish-type of the liquid white resin, resulting whitening.

We apply (paint in any manner) in a nail-polish-type on the external surface of the tooth (which has been treated or properly prepared) liquid white resin.

In this invention, which is the whitening of teeth through the painting with liquid resin, it must be noted that, there are no commercially available nail-polish-type, white, liquid resins, nor any similar white liquid products because their only existing reason is tooth whitening.

### Stage 3 Photopolymerization of the liquid white resin of Stage 2

We photopolymerize the liquid white resin with which we have painted the external surface of the tooth in Stage 2.

The synthetic resin can be substituted by by the combination of the synthetic resin with a glass ionomer material in a liquid, white, nail-polish-type final form.

## Claims

1. Method of whitening teeth, comprising the steps of:
a) treating the external surface of the teeth such as cleaning the teeth,
b) passing an isolating strip and etching the dental enamel of the teeth,
c) applying an adhesive to the external surface of the teeth,
d) applying a liquid, white, nail-polish type synthetic resin to the external surface of the teeth,
e) photo-polymerizing the resin applied to the external surface of the teeth.

2. Method of whitening teeth according to claim 1, **characterized in that** the resin comprises a glass ionomer material.

## Patentansprüche

1. Verfahren zum Bleichen von Zähnen, aufweisend die Schritte:
a) Behandeln der äußeren Oberfläche der Zähne, wie etwa Reinigen der Zähne,
b) Verlegen eines isolierenden Streifens und Ätzen des Zahnschmelzes der Zähne,
c) Auftragen eines Haftmittels auf die äußere Oberfläche der Zähne,
d) Aufbringen eines flüssigen, weißen synthetischen Harzes vom Nagelpoliertyp auf die äußere Oberfläche der Zähne,
e) Fotopolymerisieren des auf der äußeren Oberfläche der Zähne aufgebrachten Harzes.

2. Verfahren des Bleichens von Zähnen gemäß Anspruch 1, **dadurch gekennzeichnet, dass** das Harz ein Glas-Ionomer-Material umfasst.

## Revendications

1. Procédé de blanchiment des dents comprenant les étapes consistant à :
a) traiter la surface externe des dents tel que nettoyer les dents,
b) passer une bande isolante et attaquer chimiquement l'émail dentaire des dents,
c) appliquer un adhésif sur la surface externe des dents,
d) appliquer une résine synthétique, de type vernis à ongle blanc liquide sur la surface externe des dents,
e) photopolymériser la résine appliquée sur la surface externe des dents.

2. Procédé de blanchiment des dents selon la revendication 1, **caractérisé en ce que** la résine comprend un matériau ionomère vitreux.
